# EUROPEAN PATENT APPLICATION

(11) **EP 4 252 669 A1**
(43) Date of publication of application: **04.10.2023**
(21) Application number: 22165392.6
(22) Date of filing: 30.03.2022
(51) Int. Cl.: A61B 8/06, A61B 8/00, A61B 8/08

(54) **FLOW MEASUREMENT**

(71) Applicant: Koninklijke Philips N.V., 5656 AG Eindhoven (NL)
(72) Inventor: DIRKSEN, Peter, Eindhoven (NL); HAARTSEN, Jaap Roger, Eindhoven (NL); KLOOTWIJK, Johan Hendrik, Eindhoven (NL); VAN RENS, Antonia Cornelia, Eindhoven (NL)
(74) Representative: Philips Intellectual Property & Standards

(57) **Abstract**

The present invention relates to flow measurement. In order to provide improved flow measurement, a sensor (10) for flow measurement is provided that comprises a transducer arrangement (12) with at least one first transducer component (14) and at least one second transducer component (16). The at least one first transducer component and the at least one second transducer component are arranged with an at least partly common field of view in operation. The at least one first transducer component and the at least one second transducer component are configured to be operated with a phase shift.

## Description

### FIELD OF THE INVENTION

The present invention relates to flow measurement, and relates in particular to a sensor for flow measurement, to a flow measurement device, to an ultrasound imaging system for flow measurement, to a driving circuit for operating a sensor for flow measurement and to a method for flow measurement.

### BACKGROUND OF THE INVENTION

To measure blood flow in a vessel, devices such as flow wires are used, comprising a miniature piezoelectric transducer at the distal end of an elongate body. Based on the Doppler ultrasound principle, the flow wire provides blood flow velocity based on intravascular measurements. The measured Doppler frequency shift is converted into the blood velocity distribution in the arteries. The miniature piezoelectric element is connected by a long wire to the console. However, it has been shown that effects like a low signal to noise ratio (SNR), a poor bandwidth and resonance frequency variability may result in unsatisfactory measurement quality. There is a need to provide flow measurement with improved accuracy.

### SUMMARY OF THE INVENTION

The object of the present invention is solved by the subject-matter of the independent claims; further embodiments are incorporated in the dependent claims. It should be noted that the following described aspects of the invention apply also for the sensor for flow measurement, for the flow measurement device, for the ultrasound imaging system for flow measurement, for the driving circuit for operating a sensor for flow measurement and for the method for flow measurement.

According to the present invention, a sensor for flow measurement is provided. The sensor comprises a transducer arrangement with at least one first transducer component and at least one second transducer component. The at least one first transducer component and the at least one second transducer component are arranged with an at least partly common field of view in operation. The at least one first transducer component and the at least one second transducer component are configured to be operated with a phase shift.

As an effect, an ultrasound transducer with an increased opening angle, i.e. increased viewing angle or increased field of view is provided. For example, the measured volume, and thus the measured velocity profile across the vessel, in a blood flow is larger.

The increased opening angle, also referred to as acceptance angle, is suitable for large diameter vessels. While with a single transducer, due to diffraction effects, the acceptance angle may be e.g. about 25° to 30°, the increased filed of view of according to the invention provides advantages in that, like in applications having a large vessel diameter, a flow wire or other device the sensor is attached to, it is prevented that the measured flow velocity is based predominantly on measurement that includes vessel wall in the field of view or imaging path of the ultrasound transducer element and/or the blood flow velocity value measured is less dependent on the blood flow profile across the cross section of the vessel. Thus, poor signal quality results are avoided by the larger acceptance angle of the transducer.

According to an example, the second transducer component is provided annular to the first transducer component. In an example, the first transducer component and the second transducer component are arranged in a concentric manner.

According to an example, the first transducer component is provided as a first ring-type structure, and the second transducer component is provided as a second ring-type structure.

According to an example, the first transducer component and the second transducer component are provided next to each other.

According to an example, the processor is configured to drive the at least one first transducer component and the at least one second transducer component of the transducer arrangement with an oscillating alternate current as operating voltage and with opposite biasing direct current voltages for achieving the phase shift; preferably, the phase shift results in an interference effect.

According to the present invention, a flow measurement device is provided. The device comprises a sensor according to one of the preceding examples. The device also comprises an operating structure for handling and positioning the transducer array. The operating structure comprises a distal end and a proximal end. The transducer is attached to the distal end of the operating structure. Further, a data connection is provided at the proximal portion, the data connection being configured to provide measured flow data.

According to the present invention, an ultrasound imaging system for flow measurement is provided. In an option, the system comprises a sensor for flow measurement according to one of the preceding examples. In another option, additionally or alternatively, the system comprises a flow measurement device according to one of the preceding examples. Further, an operating console is provided. The operating console is configured to operate the at least one first transducer component and at least one second transducer component of the transducer arrangement of the sensor.

According to the present invention, also a driving circuit for operating a sensor for flow measurement is provided. The driving circuit comprises a primary high voltage input for supplying an alternating electric current to at least one first ultrasound transducer element and at least one second ultrasound transducer element both acting as ultrasound transmitter. The driving circuit also comprises a secondary input for a first biasing direct current voltage and a second biasing direct current voltage. The driving circuit further comprises a common-line connecting interface with a common connection for the at least one first ultrasound transducer element and the at least one second ultrasound transducer element. The driving circuit furthermore comprises a dual-line connecting interface with a first connection for connection with the at least one first ultrasound transducer element, and a second connection for connection with the at least one second ultrasound transducer element. The driving circuit comprises a signal output for providing a signal generated by the at least one first ultrasound transducer element and the at least one second ultrasound transducer element both acting as ultrasound receiver. The primary high voltage input is switchably connectable to the at least one first ultrasound transducer element and the at least one second ultrasound transducer element. The at least one first ultrasound transducer element and the at least one second ultrasound transducer element are switchably connectable to the signal output. The first biasing voltage is supplied to the first connection, and the second biasing voltage is supplied to the second connection such that at least a phase shift is provided between the at least one first ultrasound transducer element and the at least one second ultrasound transducer element.

According to an example, the primary high voltage input comprises a first high voltage input for supplying the at least one first ultrasound transducer element via the first connection, and a second high voltage input for supplying the at least one second ultrasound transducer element via the second connection. Further, at least one of the first biasing voltage and the second biasing voltage is adjustable to adapt a degree of phase-shift between the at least one first ultrasound transducer element and the at least one second ultrasound transducer element.

According to an example, the primary high voltage input is connectable to the at least one first ultrasound transducer element and the at least one second ultrasound transducer element via the common-line. Further, the first and second biasing voltages are provided as opposite bias voltages.

According to the present invention, a method for flow measurement is provided. The method comprises the following steps: in a first mode, it is provided a supplying at least one first transducer component and at least one second transducer component of a transducer arrangement with an alternating electric supply current. The at least one first transducer component and the at least one second transducer component are arranged with an at least partly common field of view in operation. It is further provided an operating of the at least one first transducer component and the at least one second transducer component with a phase shift by providing a first biasing direct current voltage to the at least one first transducer component and a second biasing direct current voltage to the at least one second transducer component.

Further, in an alternating manner in a second mode, it is provided the step of receiving signals from the at least one first transducer component and the at least one second transducer component.

According to an aspect, a flow measurement sensor is provided that comprises a first and a second transducer part that are operated with a phase shift to provide an enlarged viewing angle. In an example, a double-donut structure is provided.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a sensor for flow measurement.
Fig. 2 shows a further example of a sensor with a double donut structure.
Fig. 3 shows a cross-section through a sensor with a double donut structure.
Fig. 4 schematically shows an example of a flow measurement device.
Fig. 5 shows a flow measurement device in a vessel.
Fig. 6 shows an example of an ultrasound imaging system for flow measurement.
Fig. 7 indicates an acceptance angle of a standard donut shape membrane.
Fig. 8 indicates an acceptance angle of a double donut shaped membrane.
Fig. 9 shows a first example of a driving circuit to operate the two donuts in anti-phase (180° phase delay) or a selectable phase delay between 0° and 180°.
Fig. 10 indicates a second example of a driving circuit to operate the two donuts in anti-phase (180° phase delay) by operating the two donuts at opposite bias voltage polarity.
Fig. 11 shows basic steps of an example of a method for flow measurement.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

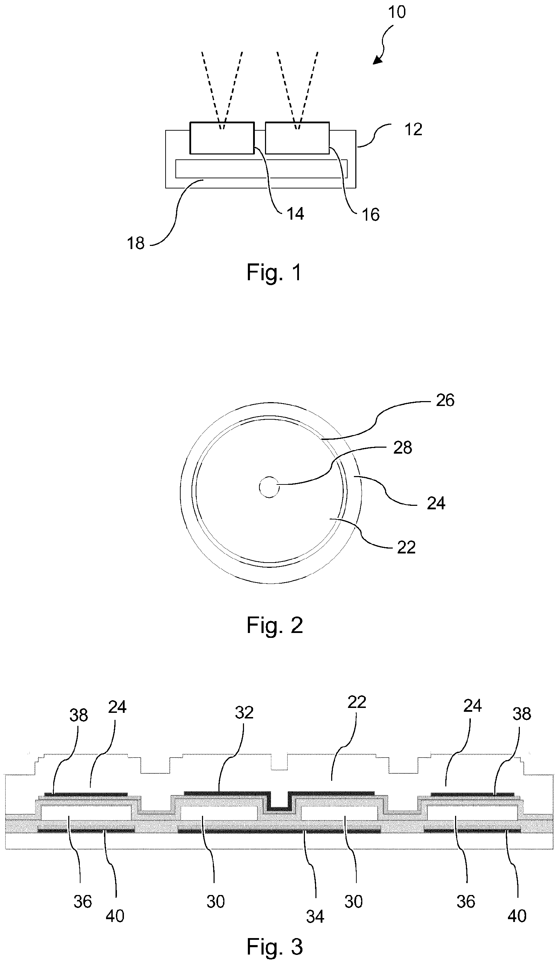

Fig. 1 schematically shows an example of a sensor 10 for flow measurement. The sensor 10 comprises a transducer arrangement 12 with at least one first transducer component 14 and at least one second transducer component 16. The at least one first transducer component 14 and the at least one second transducer component 16 are arranged with an at least partly common field of view in operation. The at least one first transducer component 14 and the at least one second transducer component 16 are configured to be operated with a phase shift.

The term "transducer component" relates to a transducer element capable of transmitting and receiving ultrasound waves.

In an example the second transducer component is arranged annular to the first transducer component. In an option, the first transducer component 14 and the second transducer component 16 are arranged in a concentric manner. The term "concentric" relates to, for example, an arrangement with overlapping geometric centers.

In an option, the first transducer component 14 is provided as a first ring-type structure; and the second transducer component 16 is provided as a second ring-type structure. The term "ring-type" relates to circular ring forms, but also to oval, square, rectangular and polygonal ring forms.

Fig. 2 shows a further example of the sensor 10 with a double donut structure. As an example, the first and the second ring-type structures are provided as a double donut-shaped structure, wherein, in relation to the first ring-type structure, e.g. a first donut 22, the second ring-type structure, e.g. a second donut 24, has a larger outer diameter surrounding a central portion 26. The first donut may also surround a central portion 28.

Fig. 3 shows a cross-section through a sensor with a double donut structure. In the cross-section, the first donut 22 is arranged in the central portion, surrounded on both sides by sections of the second donut 24. The first donut 22, i.e. the first transducer, is provided with a first ring-shaped cavity 30 and with an upper electrode 32 and a lower electrode 34. The second donut 24, i.e. the second transducer, is provided with a second ring-shaped cavity 36 and with an upper electrode 38 and a lower electrode 40. The double donuts can be manufactured with semiconductor technology, e.g. CMOS.

One aspect of the present invention is the introduction of the double donut transducer concept which is unique to capacitive micromachined ultrasound transducers (CMUT). The two donuts can be operated in anti-phase, i.e. a phase delay of 180° to achieve a very large acceptance angle > 60°. In addition, the acceptance angle can be tuned by selecting a phase delay between 0° and 180°.

Additionally, the implementation of the double donut concept in CMUT technology solves or reduces the problems related to a low SNR, poor bandwidth, reliability and performance issues, and resonance frequency drift and variability.

As an example, an inner donut with an outer diameter of 270 microns and an inner diameter of 35 microns is provided. The outer donut is respectively larger, for example with a diameter of 355 microns. An operation frequency of 12 MHz is provided.

As an advantage of a transducer provided as CMUT, in contrast to a transducer provided as lead zirconate titanate (PZT) transducer, there is no need of frequency tuning.

In an option, not shown in detail, the first transducer component 14 and the second transducer component 16 are provided next to each other. The term "next to each other" relates to an arrangement side by side, i.e. in an adjacent or adjoining manner.

In another option, also not shown in detail, the transducer arrangement 12 comprises a transducer array with a plurality of the first and the second ring-type structures.

In another option, also not shown in detail, the transducer arrangement 12 comprises a transducer array with a plurality of the first and second transducer components 14, 16 that are provided next to each other. The term "array" relates to a plurality of transducer elements.

In an example, shown in Fig. 1 as an option, it is also provided a processor 18 configured to drive and read out the transducer arrangement 12 with an operation frequency of preferably 12 MHz. The processor 18 provides the phase shift for operation of the at least one first transducer component 14 and the at least one second transducer component 16 of the transducer arrangement 12. For example, the processor 18 is provided as an application specific integrated circuit (ASIC).

In an example, the processor 18 is configured to drive the at least one first transducer component 14 and the at least one second transducer component 16 of the transducer arrangement 12 with an oscillating alternate current as operating voltage and with opposite biasing direct current voltages for achieving the phase shift.

In an option, the phase shift results in an interference effect.

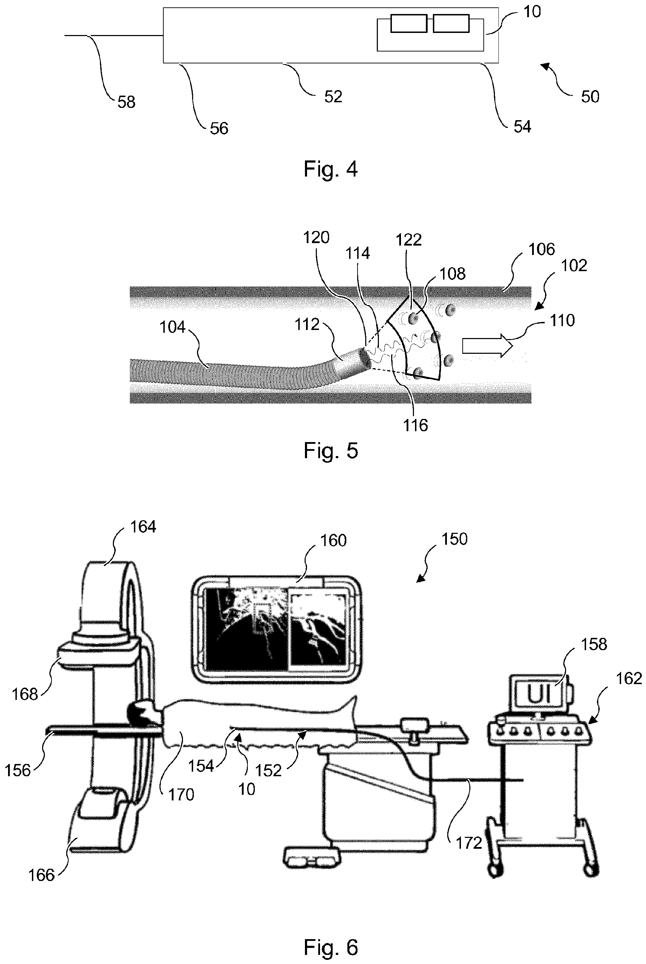

Fig. 4 schematically shows an example of a flow measurement device 50. The flow measurement device 50 comprises an example of the sensor 10 according to one of the preceding examples. Further, an operating structure 52 for handling and positioning the transducer array is provided. The operating structure 52 comprises a distal end 54 and a proximal end 56. The transducer is attached to the distal end 54 of the operating structure 52. A data connection 58 is provided at the proximal portion or the proximal end 56, the data connection 58 being configured to provide measured flow data.

The term "operating structure" relates to a physical structure that allows handling, e.g. manual handling by a user. The operating structure 52 can be a housing with portions acting as grip or handle portion. The operating structure 52 can be an elongate structure for inserting into lumen structures of a subject.

The term "distal end" relates to an end of a structure that points away from the user when operating the sensor, i.e. an end that is further away from the user and closer to the subject under examination or observation. The term "proximal end" relates to an end of a structure that points towards the user when operating the sensor, i.e. an end that is closer to the user.

For example in case of a double donut structure, the polarity of the outer ring is reversed with respect to the inner ring by adding a 180° delay to the transmitter of the outer ring.

It is also provided as an option, to tune the acceptance angle by selecting any delay between 0° and 180°.

In an option, the operating structure 52 is an elongated structure configured for inserting at least the distal end into a lumen for intrabody vascular flow measurement. For example, an intravascular device is provided. The term "intrabody" relates to flow measurement inside a body structure, e.g. inside a vessel or lumen of an organ.

Fig. 5 schematically shows a cross section through a vessel 102 with a flow measurement device 104. The vessel 102 is indicated by vessel walls 106. Blood flow inside the vessel is indicated by blood cells 108 that move along the vessel in a blood flow direction 110. The blood flow measurement device, e.g. ultrasound device 104 comprises a transducer 112 at its distal end. The transducer 112 emits ultrasound waves 114 and receives reflected waves 116 such that ultrasound measurement data is generated and forwarded to a processor (not shown). Fig. 5 also indicates an example for a field of view 120 of the transducer with a resulting sample volume 122 in which the flow of blood is detected, i.e. measured.

In an example, the flow measurement device is a flow measurement wire.

In another example, the flow measurement device is an intravascular device. In another example, the flow measurement device is an intraluminal device.

In another example, the flow measurement device is a guidewire with a flow measurement sensor.

The distal end is equipped with the flow sensor and is configured for inserting into a lumen, like a hollow organ or a vessel in a region of interest of a body structure, e.g. of a subject.

In another option, not shown in detail, the operating structure is an ultrasound probe configured for extracorporeal positioning for external measurement of flow inside a body lumen. The term "extracorporeal" relates to arranging the sensor outside a body structure.

Fig. 6 shows an example of an ultrasound imaging system 150 for flow measurement. The system 150 comprises an example of the device for flow measurement according to one of the preceding and following examples. Further, an interventional device 152 with an ultrasound transducer 154 attached to a distal portion of the interventional device 152 is provided. The interventional device 152 is at least data-connected to the device 10 for flow measurement. The ultrasound transducer 154 generates the plurality of ultrasound signals, which are provided to the data input (not shown in detail). As an option, Fig. 6 shows a subject support 156, e.g. a patient table.

The device for flow measurement may be provided as a movable control console or operating console 162 and may be equipped with a graphical user interface 158 like a display and/or audio user interface and further control elements. Still further, a monitor arrangement 160 is provided in the vicinity of the patient support 156, such as a ceiling- or wall-mounted display. As a further option, additional imaging systems are provided, like an X-ray imaging system 164 with an X-ray source 166 and an X-ray detector 168 mounted to a movably supported C-arm structure. An object of interest, like a subject 170, can be arranged on the subject support 156. The subject 170 is shown with cover drapes. A line 172 indicates the data-connection, which can be wired and/or wireless.

The operating console 162 is configured to operate the at least one first transducer component and at least one second transducer component of the transducer arrangement of the sensor. The term "operating console" relates to an interface provided for the user to control the operation of the sensor.

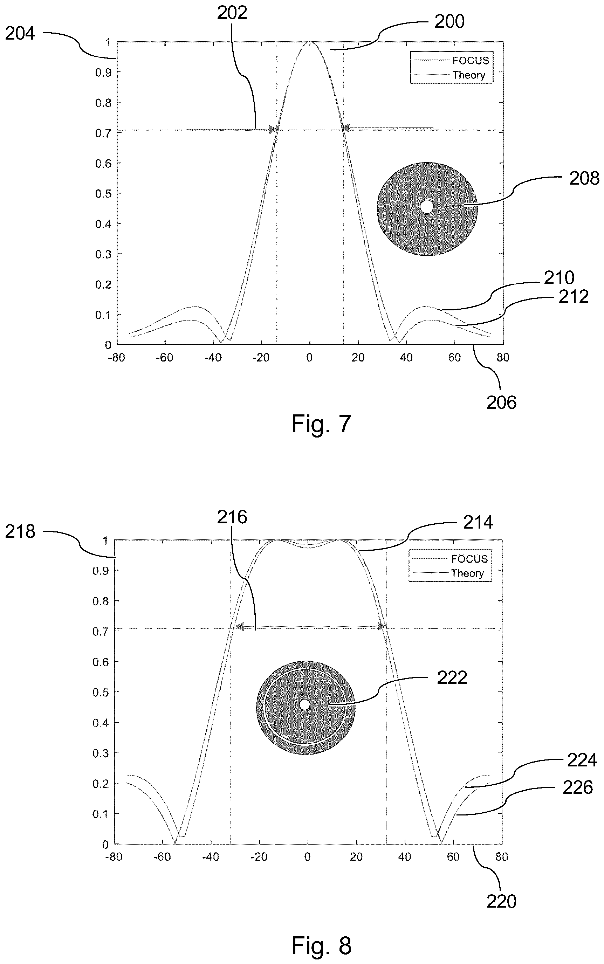

Fig. 7 indicates an acceptance angle 200 of a standard donut shape membrane, indicated by a first pair of arrows 202. A vertical axis 204 indicates e.g. normalized pressure and a horizontal axis 206 indicates the angle. A single-donut 208 is indicated as reference. A first curve 210 indicates a theoretically determined directivity function. A second curve 212 indicates a simulated directivity function.

Fig. 8 indicates an acceptance angle 214 of a double donut shape membrane, indicated by a second pair of arrows 216. A vertical axis 218 indicates e.g. normalized pressure and a horizontal axis 220 indicates the angle. A double-donut 222 is indicated as reference. A third curve 224 indicates a theoretically determined directivity function for the double-donut structure. A fourth curve 226 indicates a simulated directivity function for the double-donut structure.

As can be seen, the acceptance angle of a double donut shape is significantly larger than the acceptance angle of a single donut shape. For example, the acceptance angle of a single donut structure, for an approximately 0,7 norm pressure index, is 27°; whereas the acceptance angle of a double donut structure, for a similar approximately 0,7 norm pressure index, is 50° to 70°, e.g. 64°degrees, with a phase delay in operation of 180°.

Hence, in an example, a double donut shaped CMUT membrane is provided, see also Fig. 2 and Fig. **3Error! Reference source not found..** By adding an outer ring (donut) to a standard donut shaped CMUT membrane and operating the outer ring in opposite phase as the inner donut, a large acceptance angle can be achieved (see Fig. 7 and Fig.

### 8Error! Reference source not found.).

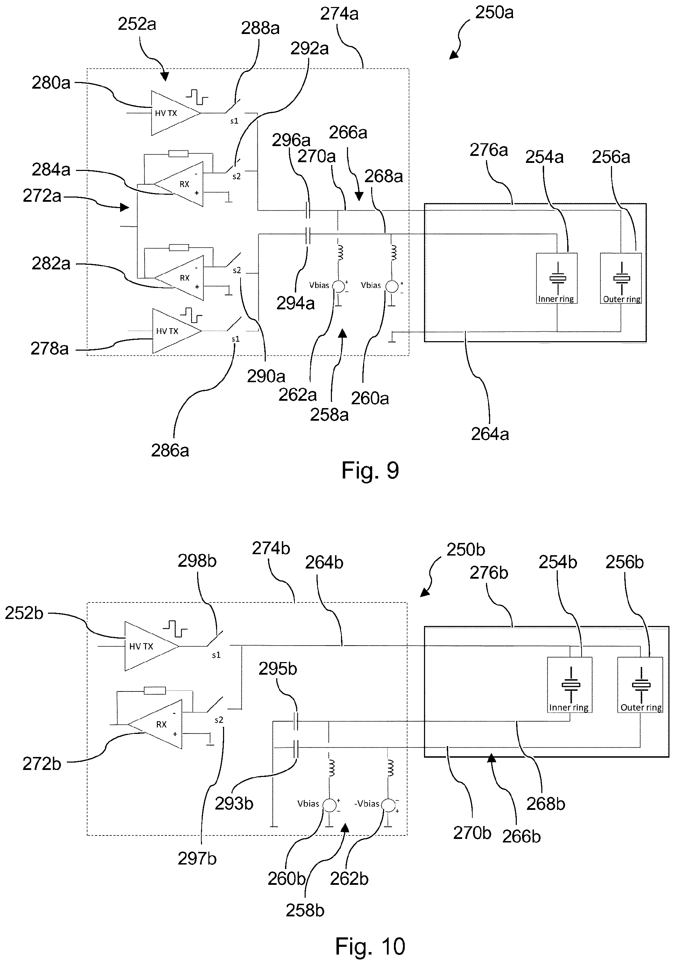

Further, as shown in Fig. 9 and Fig. 10, a driving circuit 250a and 250b for operating a sensor for flow measurement is provided. Referring to Fig. 9, the driving circuit 250a comprises a primary high voltage input 252a for supplying an alternating electric current to at least one first ultrasound transducer element 254a and at least one second ultrasound transducer element 256a both acting as ultrasound transmitter. As an example, the at least one first ultrasound transducer element 254a is provided as an inner ring of a double donut structure, and the at least one second ultrasound transducer element 256a is provided as an outer ring of a double donut structure. The driving circuit 250a comprises a secondary input 258a for a first biasing direct current voltage 260a and a second biasing direct current voltage 262a. The driving circuit 250a also comprises a common-line 264a connecting interface with a common connection for the at least one first ultrasound transducer element 254a and the at least one second ultrasound transducer element 256a. The driving circuit 250a comprises a dual-line connecting interface 266a with a first connection 268a for connection with the at least one first ultrasound transducer element 254a, and with a second connection 270a for connection with the at least one second ultrasound transducer element 256a. The driving circuit 250a comprises a signal output 272a for providing a signal generated by the at least one first ultrasound transducer element 254a and the at least one second ultrasound transducer element 256, both acting as ultrasound receiver.

In an option, the signal output 272a is split and the two output signals are processed individually.

The primary high voltage input 252a is switchably connectable to the at least one first ultrasound transducer element 254a and the at least one second ultrasound transducer element 256a. The at least one first ultrasound transducer element 254a and the at least one second ultrasound transducer element 256a are switchably connectable to the signal output 272a. The first biasing voltage 260a is supplied to the first connection 268a, and the second biasing voltage 262a is supplied to the second connection 270a such that at least a phase shift is provided between the at least one first ultrasound transducer element 254a and the at least one second ultrasound transducer element 256a.

Thus, a circuit is provided that e.g. drives the two transducer elements in anti-phase.

The term "common-line" 264a relates to a single connection supplying both transducer elements. The term "dual-line" 266a relates to two separate connections.

A first frame 274a indicates a housing structure or the like for the driving circuit 250a. A second frame 276a indicates a structure of a sensor arrangement, e.g. a structure of a flow measurement device such as an intravascular device. The driving circuit 250a can be arranged separate to the structure of the sensor arrangement. In another option, the driving circuit 250a is arranged in an integrated manner with the structure of the sensor arrangement.

The features described above are indicated with their reference number with index "a" for the option shown as example in Fig. 9, whereas similar features apply with a respective index "b" for the option shown as example in Fig. 10. Further details, in particular differing features of Fig. 9 and Fig. 10, are also described below.

In an option, the driving circuit 250a (250b) is provided for operating the sensor for flow measurement according to one of the preceding examples.

In another option, the driving circuit 250a (250b) is provided for operating the sensor in a flow measurement device according to one of the preceding examples.

In a further option, the driving circuit 250a (250b) is provided for operating the sensor in an ultrasound imaging system for flow measurement according to one of the preceding examples.

In an example, the first ultrasound transducer element 254a (254b) is provided as a first piezoelectric element, e.g. as a capacitive micromachined ultrasound transducer (CMUT) element. In an example, the second ultrasound transducer element 256a (256b) is provided as a second piezoelectric element, e.g. also as a CMUT element.

An example is provided based on CMUT ultrasound transducer using the concept of a double donut. The double donut concept allows for a large acceptance angle whereas the CMUT based ultrasound transducer allows for a large bandwidth and reduced resonance frequency variability.

The driving circuit 250a in Fig. 9 is proposed to drive the two donuts, as example. Two high voltage transmit (HV TX) signals can be chosen with a 180° phase delay to get a maximum acceptance angle. It should be noted that the acceptance angle can be tuned by choosing a phase delay between 0 and 180°.

Fig. 9 shows a first example of the driving circuit 250a to operate the two donuts in anti-phase (180° phase delay) or a selectable phase delay between 0° and 180°.

In an option, indicated in Fig. 9, the primary high voltage input 252a comprises a first high voltage input 278a for supplying the at least one first ultrasound transducer element 254a via the first connection 268a, and a second high voltage input 280a for supplying the at least one second ultrasound transducer element 256a via the second connection 270a. At least one of the first biasing voltage 260a and the second biasing voltage 262a is adjustable to adapt a degree of phase-shift between the at least one first ultrasound transducer element 254a and the at least one second ultrasound transducer element 256a.

In another option, also indicated in Fig. 9, the signal output 272a comprises a first signal output 282a for providing a signal generated by the at least one first ultrasound transducer element 254a. The signal output 272a also comprises a second signal output 284a for providing a signal generated by the at least one second ultrasound transducer element 256a.

The first high voltage input 278a is switchably connected to the first connection 268a via a first switch 286a. The second high voltage input 280a is switchably connected to the second connection 270a via a second switch 288a.

The first connection 268a is switchably connected to the first signal output 282a via a third switch 290a. The second connection 270a is switchably connected to the second signal output 284a via a fourth switch 292a.

A capacitor arrangement may be provided in the dual-line connecting interface 266a with a first capacitor 294a in the first connection 268a and a second capacitor 296a in the second connection 270a.

The common-line 264a in the example of Fig. 9 is connected to ground.

In an example, it is provided to reverse the polarity of the outer ring with respect the inner ring by reversing the bias voltage. With this concept, two acceptance angles can be selected, i.e. a narrow beam by selecting equal bias voltages or a wide beam by selecting opposite bias voltages. In an example, resonance frequencies and bandwidth can be optimized for receiving and transmitting by choosing individual optimal bias voltage levels for inner and outer ring in case of a double donut structure. As an example, the bias voltage of one of the transducers can also be chosen 0 V to 'switch' off e.g. the inner or outer element (or both).

As an alternative, the circuit in Fig. 10 is proposed to operate the two donuts, as example, in anti-phase. This is achieved by operating the two donuts at opposite bias voltage polarities. It should be noted that with this configuration, a small or large acceptance angle can be selected, i.e. by setting equal or opposite bias voltage polarities, respectively. It should also be noted that with this configuration, it is possible to slightly tune and match the resonance frequencies of the two donuts by adapting the individual bias voltage levels.

Fig. 10 indicates a second example of the driving circuit 250b to operate the two donuts in anti-phase (180° phase delay) by operating the two donuts at opposite bias voltage polarity.

In an option, indicated in Fig. 10, the primary high voltage 252b input is connectable to the at least one first ultrasound transducer element 254b and the at least one second ultrasound transducer element 256b via the common-line 264b. The first and second biasing voltages 260b, 262b are provided as opposite bias voltages.

The high voltage input 252b is switchably connected to the common-line 264b via a first further switch 298b. The common-line 264b is switchably connected to the signal output 272b via a second further switch 297b.

A capacitor arrangement may be provided in the dual-line connecting interface 266b with a first further capacitor 295b in the first connection 268b and a second further capacitor 293b in the second connection 270b.

The first connection 268b and the second connection 270b of the dual-line connecting interface 266b in Fig. 10 are connected to ground.

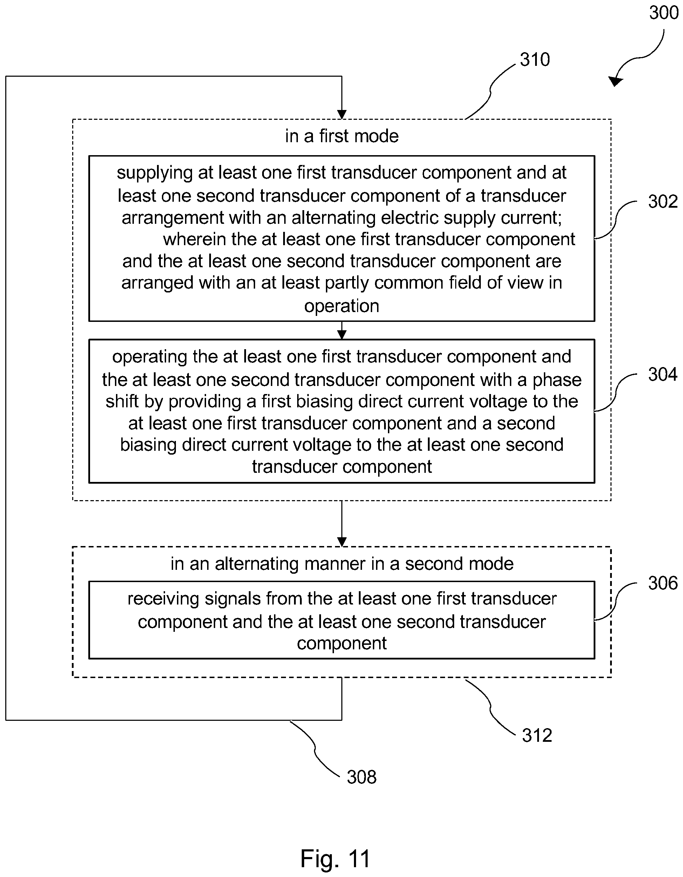

Fig. 11 shows basic steps of an example of a method 300 for flow measurement. The method comprises the following steps:
In a first mode, a supplying 302 at least one first transducer component and at least one second transducer component of a transducer arrangement with an alternating electric supply current is provided. The at least one first transducer component and the at least one second transducer component are arranged with an at least partly common field of view in operation. Further, an operating 304 of the at least one first transducer component and the at least one second transducer component with a phase shift by providing a first biasing direct current voltage to the at least one first transducer component and a second biasing direct current voltage to the at least one second transducer component is provided. The two actions, i.e. the supplying 302 with electric supply and the operating 304, are provided more or less simultaneously.

In an alternating manner in a second mode, a step of receiving 306 signals from the at least one first transducer component and the at least one second transducer component is provided.

A loop-like arrow 308 indicates an option of continuous alternating operation in the first and the second mode.

The first mode, also referred to as ultrasound sending mode, is indicated by a first frame 310. The second mode, also referred to as ultrasound receiving mode, is indicated by a second frame 312.

In an example, a computer program is provided enabling a processor to carry out the method of the preceding example.

In an example, a computer program or program element for controlling an apparatus according to one of the examples above is provided, which program or program element, when being executed by a processing unit, is adapted to perform the method steps of one of the method examples above.

In another example, a computer readable medium is provided having stored the program element of the preceding example.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A sensor (10) for flow measurement, comprising:
- a transducer arrangement (12) with at least one first transducer component (14) and at least one second transducer component (16);
wherein the at least one first transducer component and the at least one second transducer component are arranged with an at least partly common field of view in operation; and
wherein the at least one first transducer component and the at least one second transducer component are configured to be operated with a phase shift.

2. Sensor according to claim 1, wherein the second transducer component is provided annular to the first transducer component.

3. Sensor according to claim 1 or 2, wherein the first transducer component and the second transducer component are arranged in a concentric manner.

4. Sensor according to any of the preceding claims, wherein the first transducer component is provided as a first ring-type structure; and the second transducer component is provided as a second ring-type structure.

5. Sensor according to claim 1, wherein the first transducer component and the second transducer component are provided next to each other.

6. Sensor according to one of the preceding claims, wherein the transducer arrangement comprises a transducer array with:
i) a plurality of the first and the second ring-type structures; or
ii) a plurality of the first and second transducer components provided next to each other.

7. Sensor according to one of the preceding claims, wherein a processor (18) is further provided, configured to drive and/or read out the transducer arrangement; and
wherein the processor is configured to provide the phase shift for operation of the at least one first transducer component and the at least one second transducer component of the transducer arrangement.

8. Sensor according to claim 7, wherein the processor is configured to drive the at least one first transducer component and the at least one second transducer component of the transducer arrangement with an oscillating alternate current as operating voltage and with opposite biasing direct current voltages for achieving the phase shift.

9. A flow measurement device (50), comprising:
a sensor (10) according to one of the preceding claims;
an operating structure (52) for handling and positioning the transducer arrangement;
wherein the transducer arrangement is attached to the distal end of the operating structure; and
wherein a data connection (58) is provided at a proximal portion of the operating structure, the data connection being configured to provide measured flow data.

10. Device according to claim 9, wherein the operating structure is one of:
an elongated structure configured for insertion of at least the distal end into a lumen for intrabody vascular flow measurement;
an ultrasound probe configured for extracorporeal positioning for external measurement of flow inside a body lumen.

11. An ultrasound system (150) for flow measurement, comprising:
a flow measurement device according to claim 9 or 10; and
an operating console (162);
wherein the operating console is configured to operate the at least one first transducer component and at least one second transducer component of the transducer arrangement of the sensor.

12. A driving circuit (250) for operating a sensor according to any of the claims 1 to 8 for flow measurement, the driving circuit comprising:
a primary high voltage input (252) configured for supplying an alternating electric current to at least one first ultrasound transducer component and at least one second ultrasound transducer component, in transmitting mode;
a secondary input (258) for a first biasing direct current voltage and a second biasing direct current voltage;
a common-line (264) connecting interface with a common connection for the at least one first ultrasound transducer component and the at least one second ultrasound transducer component; and
a dual-line connecting interface (266) with:
a first connection (268) for connection with the at least one first ultrasound transducer component; and
a second connection (270) for connection with the at least one second ultrasound transducer component;
a signal output (272) for providing a signal generated by the at least one first ultrasound transducer component and the at least one second ultrasound transducer component, in receiving mode;
wherein the primary high voltage input is switchably connectable to the at least one first ultrasound transducer component and the at least one second ultrasound transducer component;
wherein the at least one first ultrasound transducer component and the at least one second ultrasound transducer component are switchably connectable to the signal output; and wherein the first biasing voltage is supplied to the first connection, and the second biasing voltage is supplied to the second connection such that at least a phase shift is provided between the at least one first ultrasound transducer component and the at least one second ultrasound transducer component.

13. Driving circuit according to claim 12, wherein the primary high voltage input comprises a first high voltage input (278a) for supplying the at least one first ultrasound transducer component via the first connection, and a second high voltage input (280a) for supplying the at least one second ultrasound transducer component via the second connection; and
wherein at least one of the first biasing voltage and the second biasing voltage is adjustable to adapt a degree of phase-shift between the at least one first ultrasound transducer component and the at least one second ultrasound transducer component.

14. Driving circuit according to claim 12 or 13, wherein the primary high voltage input is connectable to the at least one first ultrasound transducer component and the at least one second ultrasound transducer component via the common-line; and
wherein the first and second biasing voltages are provided as opposite bias voltages.

15. A method (300) of flow measurement, comprising the following steps:
in a first mode (310):
supplying (302) at least one first transducer component and at least one second transducer component of a transducer arrangement with an alternating electric supply current;
wherein the at least one first transducer component and the at least one second transducer component are arranged with an at least partly common field of view in operation; and
operating (304) the at least one first transducer component and the at least one second transducer component with a phase shift by providing a first biasing direct current voltage to the at least one first transducer component and a second biasing direct current voltage to the at least one second transducer component; and
in an alternating manner in a second mode (312):
receiving (306) signals from the at least one first transducer component and the at least one second transducer component.
